# EUROPEAN PATENT SPECIFICATION

(11) **EP 4 338 678 B1**
(45) Date of publication and mention of the grant of the patent: **30.04.2025**
(21) Application number: 22195649.3
(22) Date of filing: 14.09.2022
(51) Int. Cl.: A61B 8/00, A61B 8/08, G01S 15/89, G01N 29/00

(54) **FLEXIBLE ULTRASOUND TRANSDUCER**
FLEXIBLER ULTRASCHALLWANDLER
TRANSDUCTEUR ULTRASONORE FLEXIBLE

(43) Date of publication of application: 20.03.2024
(73) Proprietor: Katholieke Universiteit Leuven, 3000 Vlaams-Brabant Leuven (BE)
(72) Inventor: D'HOOGE, Jan, 2800 Mechelen (BE); INGRAM, Marcus, 3110 Rotselaar (BE)
(74) Representative: Winger

(56) References cited:
- NODA TAKUMI ET AL: "Self-shape estimation algorithm for flexible ultrasonic transducer array probe by minimizing entropy of reconstructed image", 2019 IEEE INTERNATIONAL ULTRASONICS SYMPOSIUM (IUS), IEEE, 6 October 2019 (2019-10-06), pages 131 - 134, XP033671636, DOI: 10.1109/ULTSYM.2019.8926295
- CRUZA JORGE F ET AL: "Real time autofocusing hardware for ultrasonic imaging with interfaces", 2015 IEEE INTERNATIONAL ULTRASONICS SYMPOSIUM (IUS), IEEE, 21 October 2015 (2015-10-21), pages 1 - 4, XP032799335, DOI: 10.1109/ULTSYM.2015.0544
- HUNTER A J ET AL: "Autofocusing ultrasonic imagery for non-destructive testing and evaluation of specimens with complicated geometries", NDT&E INTERNATIONAL, ELSEVIER, AMSTERDAM, NL, vol. 43, no. 2, 1 March 2010 (2010-03-01), pages 78 - 85, XP026833879, ISSN: 0963-8695, [retrieved on 20090916]

## Description

### Technical field of the invention

The present invention relates to an ultrasound imaging system comprising a flexible ultrasound device and a shape estimation module adapted to estimate the actual shape of the flexible ultrasound device, and to a method for estimating the actual shape of the flexible ultrasound device.

### Background of the invention

Ultrasound echography is one of the most used diagnostic imaging techniques as it is real-time, safe, low-cost and non-invasive. Conventional ultrasound imaging is usually performed by scanning a medium using sequentially focused beams, each firing allowing the reconstruction of one line of the final image. Data received by a receiver is commonly called pre-beamformed data, and the reconstructed image based on received data is commonly called a beamformed signal or image.

Flexible ultrasound transducer arrays provide a mean to conform the shape of an ultrasound transducer to that of the surface of an object under investigation. Such ultrasound transducers may facilitate the transmission of ultrasound waves from a transmitter, like a probe, to a load because they can be directly coupled to the surface in question. However, effectively focusing the transmitted beam while conforming the ultrasound transducer array shape to the surface of the object requires the position or location of the transducer elements to be accurately known.

Flexible ultrasound transducer arrays facilitate imaging through complex surfaces that were not previously accessible in either the medical or non-destructive testing (NDT) domains using standard array transducers. In the medical domain, imaging through curved surfaces such as the chest or thorax typically requires pressing the rigid transducer against the tissue to force the tissue to conform to the flat shape of the transducer. As such, smaller apertures are typically deployed in medical applications that in turn limits the field of view. In the NDT community, the solution has been to immerse the steel target and array transducer in water and to deploy advanced beamforming strategies to account for refraction at the acoustic interface between the two.

Where the array element positions are consistent during a given acquisition cycle, additional hardware can be coupled to the ultrasound array to determine the relative element positions. Existing solutions, not necessarily in the medical field, provide (or even require) certainty in the element positions and facilitate the transmission of ultrasound in the target but do not provide a robust solution where variation in the element positions of the transducer array may occur. Recent advances in microelectronics indicate that such flexible arrays can be implemented as wearable ultrasound sensors for medical applications. In this case, incorporating additional hardware for array shape estimation creates a burden on the patient as the device becomes too bulky to become wearable itself.

It is possible to estimate the array element positions from artefacts relating to a spatial point of reference located inside the image target such as a point scatterer or plane reflector. Shape estimation in NDT applications has also been proposed by iterating the element positions over a range of shapes and using the shape that maximised the pixel intensity as an indication of the correct shape; however, this is susceptible to interference from other image artefacts.

Alternatively, the time of flight of an ultrasound pulse between element indices has been demonstrated as a tool for shape estimation. In that case the spatial reference point corresponds to the array itself. In such state-of-the-art algorithms a single element is used to transmit while the remaining elements receive. The time of flight of the transmitted pulse to each of the receive element indices is used to fit a polynomial corresponding to the array shape. A disadvantage of this approach is that any artefacts located along the path length between the spatial reference points can introduce uncertainty in the estimated array shape.

It is possible to estimate the array element positions from artefacts relating to a spatial point of reference located inside the image target such as a point scatterer or plane reflector. Shape estimation in NDT applications has also been proposed by iterating the element positions over a range of shapes and using the shape that maximized the pixel intensity as an indication of the correct shape; however, this is susceptible to interference from other image artefacts.

NODA, T. et al. "Self-shape estimation algorithm for flexible ultrasonic transducer array probe by minimizing entropy of reconstructed image", 6 October 2019 IEEE International Ultrasonics Symposium, pages 131-134, discloses a shape estimation algorithm for a flexible ultrasound device based on the entropy of a reconstructed image using an assumed array shape. A drawback of this is that the variation across the full image or an ROI within the image should be determined, and that the full image scene must be reconstructed for each assumed array shape, comprising the temporal resolution of the algorithm, requiring a significant amount of computing time and resources. Moreover, for echocardiographic applications the actual shape of the flexible ultrasound device, i.e. the array element positions, may vary as the patient breathes requiring a computationally faster shape estimation algorithm.

CRUZA, Jorge F.; MEDINA-VALDES, Luis; FRITSCH, Carlos. Real time autofocusing hardware for ultrasonic imaging with interfaces. In: 2015 IEEE International Ultrasonics Symposium (IUS). IEEE, 2015. p. 1-4 describes an interactive method for determining a shape metric. The method comprises obtaining a time-of-flight from every array element and scan line to two foci, taking into account refraction. HUNTER, Alan J.; DRINKWATER, Bruce W.; WILCOX, Paul D. Autofocusing ultrasonic imagery for non-destructive testing and evaluation of specimens with complicated geometries. Ndt & E International, 2010, 43.2: 78-85 describes autofocusing based on an assessment of the sharpness of an image for determining a shape metric.

Hence, a drawback of the existing solutions in both the medical and non-medical field for the estimation of an effective shape of an ultrasound transducer array require at least one spatial feature of an image to be optimized with respect to the array shape. The variation in these artefacts depends on the dynamic range and number of focal points in the image.

All the aforementioned reasons call for an ultrasound imaging system and method for at least medical purposes allowing an accurate and reliable estimation of a shape of a flexible or deformable ultrasound transducer array device, in particular to estimate the effective position of the transducer elements defining the array, wherein the computational efficiency may be optimized, fast and robust without - or to a minimal extend - loss of image accuracy and resolution.

### Summary of the invention

The current invention aims to address the aforementioned drawbacks and to provide a method for estimating an actual shape of a flexible ultrasound device, i.e. an actual shape of the transducer elements array disposed on a flexible supporting platform, without a need of an external image related reference point, and which may be implemented in real time with respect to the image reconstruction.

It is an advantage of embodiments of the present invention that an actual shape of a flexible ultrasound device can be estimated independently of any spatial reference points and can be implemented in real time with respect to the image reconstruction.

It is an advantage of embodiments of the present invention that the actual shape of a flexible transducer may be estimated by a single transmit event.

It is an advantage of embodiments of the present invention that any actual shape of a flexible transducer may be estimated.

It is an advantage of embodiments of the present invention that the method is easy-to-implement, accurate and robust. Embodiments of the present invention are not, or to a minimal extent, sensitive to image artefacts located along the path length of the ultrasound beam and is purely software based so can be easily deployed on the flexible ultrasound device itself.

It is an advantage that no additional hardware, like optical fibres, is required to be coupled to the transducer elements to measure their position or location in situ. By exploiting the echo signal phase variation across the respective receive channels, all information relating to a shape of the ultrasound flexible device may be contained within a set of received echo signals. Therefore, the image target does not need to contain specific artefacts nor does the full image need to be generated for each assumed shape of the flexible ultrasound device, i.e. each investigated array shape.

The current invention aims to address the aforementioned drawbacks and to provide a method for estimating an actual shape of a flexible ultrasound device, i.e. an actual shape of the transducer elements array disposed on a flexible supporting platform, without a need of an external image related reference point, and which may be implemented in real time with respect to the image reconstruction.

According to a first aspect, the present invention provides a method for estimating an actual shape of a flexible ultrasound device, wherein the flexible ultrasound device comprises a flexible supporting platform and a plurality of transducer elements disposed in an array configuration on the flexible supporting platform, the method comprising: transmitting, using a first portion of the plurality of transducer elements and based on an assumed shape of the flexible ultrasound device, a focussed ultrasound beam having a predetermined transmit focus position; receiving, using a second portion of the plurality of transducer elements, an echo signal responsive to the transmitted focussed ultrasound beam, wherein each transducer element of the second portion of the plurality of transducer elements is electronically connected to a respective receive channel; calculating, based on the received echo signal, a signal phase coherence image across the receive channels; and estimating, by calculating a spatial offset in the signal phase coherence image between the predetermined transmit focus position and an observed minimum phase coherence location, the actual shape of the flexible ultrasound device.

According to a particular embodiment of the present invention, the spatial offset comprises an axial offset to correct for the transducer element positions relative path length per transducer element, and a lateral offset to correct for angular coordinates of the transducer elements relative to an apex of the flexible ultrasound device.

According to a particular embodiment of the present invention, the calculated spatial offset is compared with a calibration image or calibration curve to estimate the actual shape of the flexible ultrasound device.

According to a specific embodiment of the present invention, the transmitted focussed ultrasound beams consists of a plurality of focussed ultrasound beams each having a corresponding transmit focus position, and wherein the received echo signal consists of a plurality of echo signals responsive to the transmitted plurality of focussed ultrasound beams.

According to a particular embodiment of the present invention, the method comprises a step of filtering the signal phase coherence image.

According to a particular embodiment of the present invention, the first and second portion of transducer elements are identical.

According to a particular embodiment of the present invention, the method comprises a step of quantifying the echo signal phase coherence.

According to a particular embodiment of the present invention, the plurality of beams are transmitted as parallel simultaneous beams.

According to a second aspect of the invention, there is provided an ultrasound imaging system, comprising
- a flexible ultrasound device, wherein the flexible ultrasound device comprises a flexible supporting platform and a plurality of transducer elements disposed in an array configuration on the flexible supporting platform;
- a data storage unit storing a shape estimation module and configured to store data from the shape estimation module; and
- a data processing unit connected to the flexible ultrasound device and the data storage unit, and adapted to receive data from the flexible ultrasound device and to process the received data;
   wherein, when executed, the shape estimation module performs the steps of:
   - transmitting, using a first portion of the plurality of transducer elements and based on an assumed shape of the flexible ultrasound device, a focussed ultrasound beam having a predetermined transmit focus position;
   - receiving, using a second portion of the plurality of transducer elements, an echo signal responsive to the transmitted focussed ultrasound beam, wherein each transducer element of the second portion of the plurality of transducer elements is electronically connected to a respective receive channel;
   - calculating, based on the received echo signal, a signal phase coherence image across the receive channels; and
   - estimating, by calculating a spatial offset in the signal phase coherence image between the predetermined transmit focus position and an observed minimum phase coherence location, the actual shape of the flexible ultrasound device.

According to a third aspect of the invention, there is provided a computer program comprising instructions to cause the system according to any of aforementioned embodiments to execute the steps of the method according to any of aforementioned embodiments.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 depicts a flowchart of an actual shape estimation algorithm according to embodiments of the present invention;
Fig. 2 shows a schematic illustration of signal phase coherence image comprising a predetermined transmit focus position and an observed minimum phase coherence location obtained according to embodiments of the present invention;
Fig. 3a, 3b and 3c depict a schematic illustration of signal phase coherence images according to embodiments of the present invention for multiple transmit events;
Fig. 4 shows a comparison between the actual shape of a flexible ultrasound device and an estimated shaped according to embodiments of the present invention;
Fig. 5 shows a comparison between the actual shape of a flexible ultrasound device and an estimated shaped according to embodiments of the present invention;

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B. Furthermore, the terms first, second, third and the like in the description and in the claims, are used for distinguishing between similar elements. It is to be understood that the terms so used may be interchangeable under appropriate circumstances. In the drawings, like reference numerals indicate like features; and, a reference numeral appearing in more than one figure refers to the same element. Furthermore, while some embodiments described herein include some but not other features included in other embodiments, combinations of features of different embodiments are meant to be within the scope of the invention, and form different embodiments, as would be understood by those in the art.

Unless indicated otherwise, when in the present application reference is made to "a flexible ultrasound device", reference may be made to a transducer comprising a flexible supporting platform and a plurality of transducer elements disposed in an array configuration on the flexible supporting platform. Hence, when reference is made to "a flexible ultrasound device", reference may be made to a flexible ultrasound transducer array.

Unless indicated otherwise, when reference is made to "a shape of an ultrasound transducer array", reference may be made to a shape of the flexible supporting platform on which transducer elements are disposed in an array configuration. Hence, when reference is made to "a shape of an ultrasound transducer array", reference may be made to the shape of the array expressed by shape-related parameters like the opening angle of the flexible array and the radius from which the spatial position or location, expressed in coordinates, of the transducer elements defining the array may be determined, wherein the position or location is expressed relative to a reference point of the array, like, without being limited thereto, the array apex. According to embodiments of the present invention, the coordinates defining the position or location of the transducer elements, may also be obtained directly, i.e. without prior knowledge of the opening angle and/or radius of the array.

Unless indicated otherwise, when reference is made to "an actual shape" of a flexible ultrasound device, reference may be made to the current or present shape of the flexible ultrasound device which may be estimated according to embodiments of the present invention.

Unless indicated otherwise, when reference is made to "echo signal phase variation" across receive channels, reference may be made to echo signal phase coherence across receive channels.

Where in embodiments of the present invention reference is made to a "a flexible substrate" or "flexible array", reference may be made to a re-shapeable platform adapted to bend or flex at least partly around an object following the curvature of the outer surface of the object, wherein the bending or flexing of the platform may result in a change of the shape of the platform between a substantially planar shape to a substantially convex shape.

Estimating an actual shape of a flexible transducer element, i.e. estimating the position or coordinates of the transducer elements, is of utmost importance for ultrasound image construction when dealing with ultrasound devices, in particular phased-array ultrasound transducers.

Indeed, the energy transmitted from a first portion of transducer elements of the flexible ultrasound device, having the plurality of transducer elements configured in an array configuration, is focused by applying a predetermined transmit delay time to each first portion transducer element with respect to a transducer specified reference point, like for example, without being limited thereto, the array apex. These predetermined transmit delay times may be calculated based on the predetermined or derived transducer element coordinates and the speed of sound in the load medium. When these delays are applied across the aperture, the transmitted signals, defining the transmit beam, arriving at the desired focus location are in phase resulting in constructive interference, maximising the energy intensity at the focus or focal point.

The desired focus location may also be referred to as a predetermined transmit focus position of a transmitted focussed ultrasound beams.

Contrary, if uncertainty is introduced to the transducer element coordinates applying a time delay based on the expected transducer element coordinates, then the transmitted signals arrive out of phase from one another. This spreads the total energy of the transmit beam across a larger volume as fewer signals constructively interfere at the desired position. This uncertainty translates to a defocussing or blurring of the images reconstructed from the received echo signal across the receive channel data.

The fundamental idea behind embodiments of the current invention is related to the use of a signal phase coherence image which may be calculated based on the received echo signal or signals. In order to generate a signal phase coherence image, an acquisition of the received echo signals, or echo channel data, is required similar to BMode imaging. The difference between a beamformed BMode image and a signal phase coherence lies in the manipulation or processing of the data. In both methodologies, a set of image pixels, i.e. focus positions, are set with respect to a known coordinate system, like the angle and depth measured (or expressed) from (with respect to) the array apex of the array of transducer elements of the flexible ultrasound device. At each (predetermined) focus position the time needed for an ultrasound wave to travel from the transmitting transducer element to the (predetermined) focus position and back to the array apex is determined using the Pythagoras formula. This time-of-flight is used to find the correct sample value in the channel data set for that given transmit-receive combination. In case of standard Bmode imaging, these values are merely summed for each combination. This means that the image only represents the amplitude information of the received channel data. For phase coherence imaging, the phase information is extracted from these data. There are several ways of doing this, wherein the easiest may be to encode the phase with a binary descriptor, also referred to as sign coherence imaging wherein the phase is assigned either a value of plus 1 or minus 1 depending on the sign of the received signal. The value of the image pixel is then a metric closely related to the variance of these encoded binary values, where the variance across the channels is measured.

According to a specific embodiment of the present invention, when the method steps are repeated for multiple transmit events, an average estimated array shape can be determined. To determine the array element locations, the time delays required to focus at the observed phase coherence minimum using a flat array are first determined. In order to determine the position of the transducer elements in the array with respect to a reference point, like the apex, the time delays required to focus a transmitted beam at the observed phase coherence minimum assuming a flat shaped array are first determined. Next, an ultrasound pulse is theoretically transmitted from the initially designed focus position using time delays corresponding to the observed focus position. The element locations then correspond to the theoretically position of the wavefront when it crosses the apex of the array. This process can be repeated for each transmit event resulting in a cumulative estimation of the element locations.

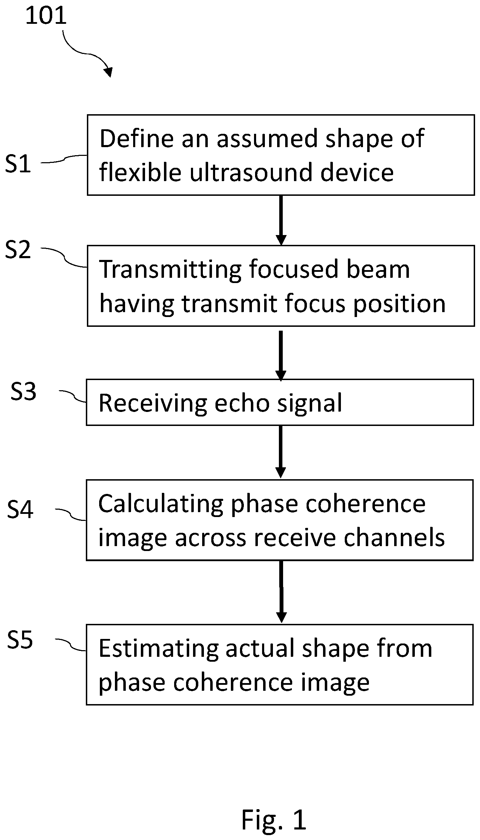

Referring to Fig. 1 a flowchart 101 of an actual shape estimation method of a flexible ultrasound device according to embodiments of the present invention is depicted. The flexible ultrasound device comprises a flexible supporting platform and a plurality of transducer elements disposed in an array configuration on the flexible supporting platform. In a first step S1, a shape of the flexible ultrasound device is defined or predetermined. According to specific embodiments of the present invention, this shape may be flat or symmetric with respect to a reference point, for example, but without limited thereto, the array apex. In a second step S2, a focused ultrasound beam is transmitted using a first portion of the plurality of transducer elements and based on the assumed shape of the flexible ultrasound device, which defines a predetermined transmit focus position. In a third step S3 an echo signal responsive to the transmitted focused ultrasound beam is received using a second portion of the plurality of transducer elements. Each transducer element of the second portion of the plurality of transducer elements is electronically connected to a respective receive channel. In a fourth step S4 a signal phase coherence image is calculated, wherein the signal phase coherence image is based on the received echo signal of step S3. Finally, in a fifth step S5, the actual shape of the flexible ultrasound device is estimated using the calculated signal phase coherence image of step S4. From the signal phase coherence image, a spatial offset is calculated between the location or position in the image of an observed minimum and the predetermined transmit focus position.

According to a specific embodiment of the present invention, step S2 may be characterized by transmitting a plurality of focused ultrasound beams wherein each corresponding with a predetermined transmit focus position. According to this embodiment, the second portion of transducer elements may receive S3 echo signals responsive to the plurality of focused ultrasound beams which may be used for the calculation S4 of the signal phase coherence image. The actual shape of the flexible ultrasound device is estimated by calculating S5 a spatial offset between the plurality of predetermined transmit focus positions and the observed minimum phase coherence location in the signal phase coherence image.

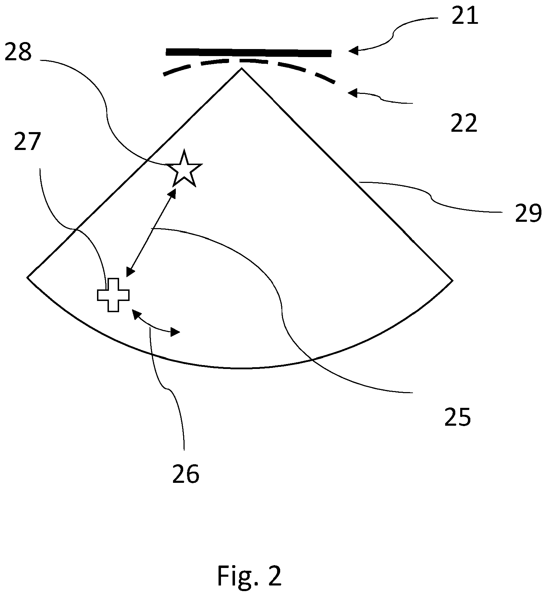

Referring to Fig. 2, a schematic illustration of signal phase coherence image 29 comprising a predetermined transmit focus position 27 and an observed minimum phase coherence location 28 is shown. The predetermined transmit focus position 27 is based on an assumed shape 21 of the flexible ultrasound device, whereas the location or position of the observed minimum 28 in the signal phase coherence image is determined by the actual shape 22 of the flexible ultrasound device.

According to embodiments of the present invention, the assumed shape 21 may be corrected to estimate the actual shape of the flexible ultrasound device by calculating the spatial offset 25, 26 between the location or position of the observed minimum phase coherence 28 and the predetermined or designed transmit focus position 27 in the signal phase coherence image 29. The axial offset component 26 of the spatial offset may be a measure to correct of the element relative path length per element, whereas the lateral offset component 25 of the spatial offset is used to correct for angular coordinates of the transducer elements relative to the apex.

According to embodiments of the present invention, the spatial offset 25, 26 may be compared with a calibration image or curve to estimate the actual shape of the flexible ultrasound device. The calibration image or curve may be stored on a computing device and called by the data processing unit to be compared with the spatial offset 25, 26.

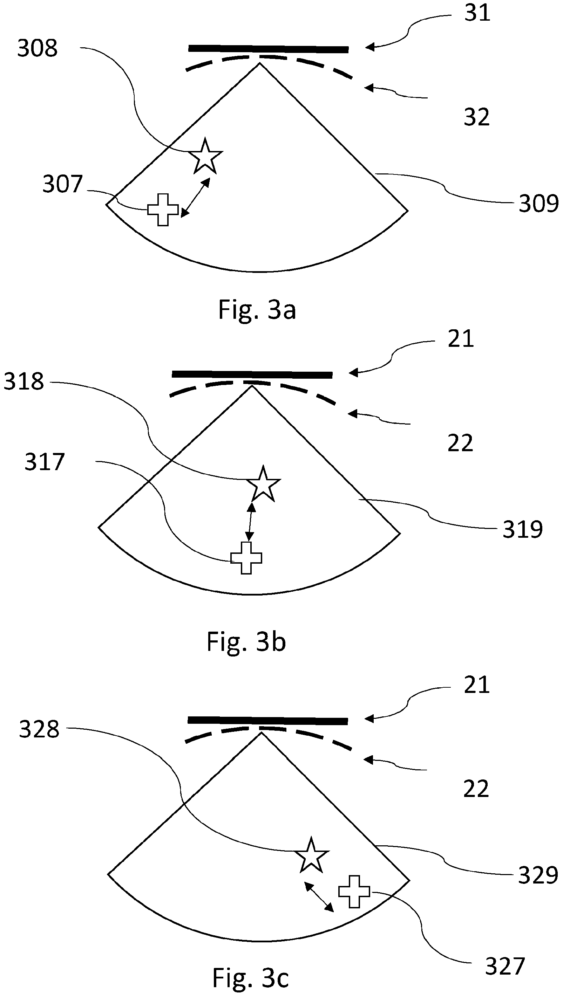

Referring to Fig. 3a, 3b and 3c, schematic illustrations of signal phase coherence images 309, 319, 329 are shown according to embodiments of the present invention for estimating an actual shape 32 of a flexible ultrasound device. The method comprises a plurality of transmitting events wherein each focused ultrasound, based on an assumed shape of the flexible ultrasound device 31, has a predetermined transmit focus position 307, 317, 327. The plurality of focused ultrasound beams are transmitted using a first portion of the plurality of transducer elements. The received echo signals responsive to the plurality of focused ultrasound beams are used to calculate a signal phase coherence image 309, 319, 329 across the receive channels. For each transmitted beam a spatial offset between the location or position of the predetermined transmit focus position 307, 317, 327 and the observed minimum phase coherence is calculated to estimate the actual shape 32 of the flexible ultrasound device.

In phase coherence imaging, multiple or single transmit events are used to spread the transmitted energy across the full image space. This means that the related transmit focus positions correspond to locations where the transmitted energy is highest, as set by the transmit time delays for each channel. However, it is also possible to artificially focus the energy at every location, thus creating a phase coherence image using only a single transmit event. In doing so, a map of the signal phase variation, hence a signal phase coherence image, in space is created for that single transmit event defining a minimum at the predetermined transmit focus position. The location or position of this phase coherence image minimum may be directly related to the applied time delays, hence the assumed or pre-defined shape of the flexible ultrasound device, to focus the transmitted beam at the predetermined transmit focus position. According to embodiments of the present invention, any variation or difference between the assumed shape and actual shape of the flexible ultrasound device will inherently shift the position or location of the observed signal phase coherence minimum. This shift of the observed minimum in comparison with the predetermined or designed transmit focus position will be used to retrospectively estimate the actual shape of the flexible ultrasound device, hence, the actual shape of the array of transducer elements. When the steps of transmitting a focused ultrasound beam based on an assumed shape of the flexible ultrasound device, receiving the responsive echo signal, calculating the signal phase coherence image and determining the spatial offset between the predetermined transmit focus position and observed minimum in the signal phase coherence image, are repeated for multiple transmit events, an average estimated shape of the flexible ultrasound transducer may be determined.

As illustrated in the previous figures, to determine the position or location of the transducer elements in the array configuration, the predetermined time delays to focus the transmitted beam at the predetermined transmit focus position may first be applied assuming a flat shape or array of transducer elements. Thereafter an ultrasound pulse or beam is transmitted from the first portion of transducer elements to the predetermined transmit focus position, using the theoretically time-of-flight from the transmitting transducer elements to the predetermined transmit focus position. Next, time delays may be applied corresponding to the observed focus position. The location or position of the transducer elements then correspond to the theoretical position or location of the wavefront when it crossed the apex of the array. These steps may be repeated for each transmit event resulting in a cumulative estimation of the transducer element locations in the array.

The aforementioned description may be applicable to embodiments of the present invention wherein spatial offset between a predetermined transmit focus position and the observed phase coherence minimum comprises an axial offset component and a lateral offset component. The axial offset component measures the relative path length per transducer element, whereas the lateral offset component is a measure to correct the angular coordinates of the transducer elements shifted about a reference point, like the apex of the flexible ultrasound device.

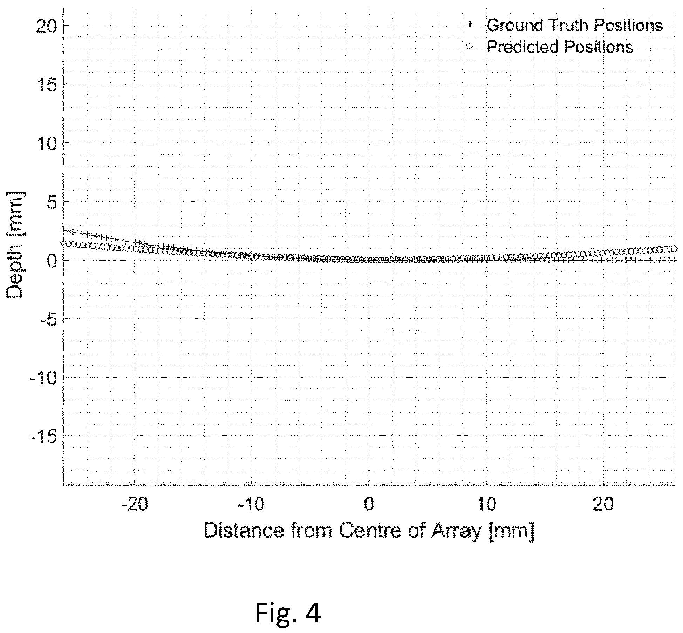

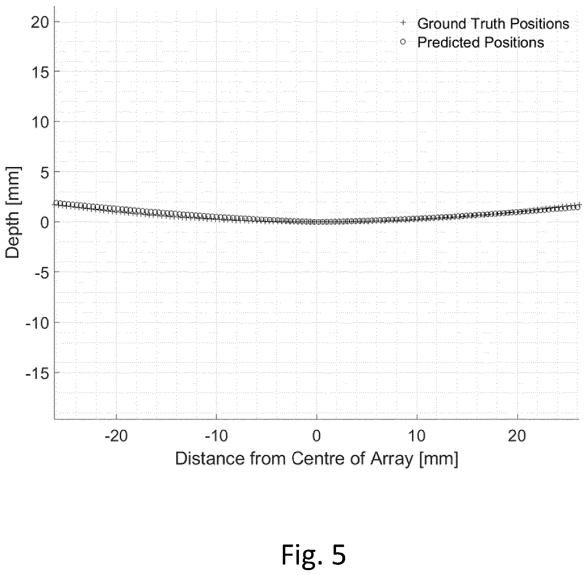

Fig. 4 and Fig. 5 show a comparison between the actual locations of the transducer elements and the estimated locations derived according to embodiments of the present invention. For these experiments, the flexible ultrasound device comprises a flexible supporting platform on which 128 transducer elements were disposed in a 1D array configuration. It can be observed that that there is significant spatial overlap between the known location (represented by crosses; ground truth positions) and the estimated locations (represented by open dots; predicted positions), which illustrates the effectiveness and high accuracy of the current invention. In these two cases the positional error is around 0.3 wavelengths. This means that the average distance between the (actual) known and estimated locations of the transducer elements, across the 128 locations, was 30% of the wavelength of the transmitted ultrasound pulse. This is significant because the wavelength is important in term of the array design, which is typically 0.5 wavelengths for phased arrays or 1.0 wavelengths for linear arrays. This means the average error is less than the distance between any two elements.

Table I provides the derived positional errors and cross correlations when applying embodiments of the present invention for different shapes of flexible ultrasound transducers. Each shape is parametrized using a symmetry parameter ("sym") and a degree of curvature parameter ("ang"). For cases that were asymmetric (sym equals to zero), this indicates that half of the array was already "flat", so half of the initial guess was already correct. This was investigated to test the sensitivity of the method to small variations in shape. However, this also biases the positional error to lower values (all below 0.4 wavelengths here). For symmetric shapes, where the positional variation was greater to start with, the error is always less than 0.8 wavelengths. Given the array had an element pitch of 0.66 wavelengths, this means the positional error is on the same order of magnitude as a the pitch. However, in terms of array transducer design, this is more important in the lateral direction than the axial and our metric does not differentiate between these two, rather it is a measure of the total positional error. In terms of cross correlation, nearly every shape investigated resulted in a higher correlation with the ground truth than when only a flat shape was assumed. The key exception here is when the array is flat. Since the maximum correlation possible is 1, any predicted shape that was not perfectly aligned would result in a lower correlation. However, this does indicate the sensitivity of the shape estimation algorithm to noise.

**Table I**

| Sym | Ang | Error (λ) | Xcorr flat | Xcorr pred |
|---|---|---|---|---|
| 1 | 0 | 0.77 | 1 | 0.76 |
| 1 | 20 | 0.42 | 0.68 | 0.86 |
| 1 | 16 | 0.45 | 0.68 | 0.74 |
| 1 | 12 | 0.59 | 0.68 | 0.81 |
| 1 | 8 | 0.74 | 0.7 | 0.84 |
| 0 | 20 | 0.31 | 0.72 | 0.75 |
| 0 | 16 | 0.19 | 0.7 | 0.82 |
| 0 | 12 | 0.37 | 0.69 | 0.69 |
| 0 | 8 | 0.3 | 0.69 | 0.81 |

## Claims

1. A method for estimating an actual shape of a flexible ultrasound device, wherein the flexible ultrasound device comprises a flexible supporting platform and a plurality of transducer elements disposed in an array configuration on the flexible supporting platform, the method comprising:
- transmitting, using a first portion of the plurality of transducer elements and based on an assumed shape of the flexible ultrasound device, a focussed ultrasound beam having a predetermined transmit focus position;
- receiving, using a second portion of the plurality of transducer elements, an echo signal responsive to the transmitted focussed ultrasound beam, wherein each transducer element of the second portion of the plurality of transducer elements is electronically connected to a respective receive channel;
- calculating, based on the received echo signal, a signal phase coherence image across the receive channels; and
- estimating, by calculating a spatial offset in the signal phase coherence image between the predetermined transmit focus position and an observed minimum phase coherence location, the actual shape of the flexible ultrasound device.

2. The method according to claim 1, wherein the spatial offset comprises an axial offset to correct for the transducer element positions relative path length per transducer element, and a lateral offset to correct for angular coordinates of the transducer elements relative to an apex of the flexible ultrasound device.

3. The method according to claim 1, wherein the calculated spatial offset is compared with a calibration image or calibration curve to estimate the actual shape of the flexible ultrasound device.

4. The method according to any of the previous claims, wherein the transmitted focussed ultrasound beams consists of a plurality of focussed ultrasound beams each having a corresponding transmit focus position, and wherein the received echo signal consists of a plurality of echo signals responsive to the transmitted plurality of focussed ultrasound beams.

5. The method according to any of the previous claims, further comprising a step of filtering the signal phase coherence image.

6. The method according to any of the previous claims, wherein the first and second portion of transducer elements are identical.

7. The method according to any of the previous claims, further comprising a step of quantifying the echo signal phase coherence.

8. The method according to claim 4, wherein the plurality of beams are transmitted as parallel simultaneous beams.

9. An ultrasound imaging system, comprising:
- a flexible ultrasound device, wherein the flexible ultrasound device comprises a flexible supporting platform and a plurality of transducer elements disposed in an array configuration on the flexible supporting platform;
- a data storage unit storing a shape estimation module and configured to store data from the shape estimation module; and
- a data processing unit connected to the flexible ultrasound device and the data storage unit, and adapted to receive data from the flexible ultrasound device and to process the received data;
wherein, when executed, the shape estimation module performs the steps of:
- transmitting, using a first portion of the plurality of transducer elements and based on an assumed shape of the flexible ultrasound device, a focussed ultrasound beam having a predetermined transmit focus position;
- receiving, using a second portion of the plurality of transducer elements, an echo signal responsive to the transmitted focussed ultrasound beam, wherein each transducer element of the second portion of the plurality of transducer elements is electronically connected to a respective receive channel;
- calculating, based on the received echo signal, a signal phase coherence image across the receive channels; and
- estimating, by calculating a spatial offset in the signal phase coherence image between the predetermined transmit focus position and an observed minimum phase coherence location, the actual shape of the flexible ultrasound device.

10. The ultrasound imaging system according to claim 9, wherein, when executing the shape estimation method, the spatial offset comprises an axial offset to correct for the element positions relative path length per element, and a lateral offset to correct for angular coordinates of the elements relative to the apex.

11. The ultrasound imaging system according to claim 9, wherein, when executing the shape estimation method, the calculated spatial offset is compared with a calibration image or curve to estimate the actual shape of the flexible ultrasound device.

12. A computer program comprising instructions to cause the system according to any of the claims 9 to 11 to execute the steps of the method according to any of the claims 1 to 8.

## Patentansprüche

1. Ein Verfahren zum Schätzen einer tatsächlichen Form einer flexiblen Ultraschallvorrichtung, wobei die flexible Ultraschallvorrichtung eine flexible Trägerplattform und eine Vielzahl von Wandlerelementen umfasst, die in einer Array-Konfiguration der flexiblen Trägerplattform angeordnet sind, wobei das Verfahren umfasst:
- Senden unter Verwendung eines ersten Abschnitts der Vielzahl von Wandlerelementen und basierend auf einer angenommenen Form der flexiblen Ultraschallvorrichtung eines fokussierten Ultraschallstrahls, der eine vorbestimmte Sendefokusposition aufweist;
- Empfangen unter Verwendung eines zweiten Abschnitts der Vielzahl von Wandlerelementen eines Echosignals als Reaktion auf den gesendeten fokussierten Ultraschallstrahl, wobei jedes Wandlerelement des zweiten Abschnitts der Vielzahl von Wandlerelementen elektronisch mit einem jeweiligen Empfangskanal verbunden ist;
- Berechnen basierend auf dem empfangenen Echosignal eines Signalphasenkohärenzbildes über die Empfangskanäle; und
- Schätzen durch Berechnen eines räumlichen Versatzes im Signalphasenkohärenzbild zwischen der vorgegebenen Sendefokusposition und einem beobachteten Ort minimaler Phasenkohärenz der tatsächlichen Form der flexiblen Ultraschallvorrichtung.

2. Das Verfahren nach Anspruch 1, wobei der räumliche Versatz einen axialen Versatz umfasst, um eine relative Weglänge der Wandlerelementpositionen je Wandlerelement zu korrigieren, und einen seitlichen Versatz, um Winkelkoordinaten der Wandlerelemente in Bezug zu einem Scheitelpunkt der flexiblen Ultraschallvorrichtung zu korrigieren.

3. Das Verfahren nach Anspruch 1, wobei der berechnete räumliche Versatz mit einem Kalibrierungsbild oder einer Kalibrierungskurve verglichen wird, um die tatsächliche Form der flexiblen Ultraschallvorrichtung zu schätzen.

4. Das Verfahren nach einem der vorstehenden Ansprüche, wobei die gesendeten fokussierten Ultraschallstrahlen aus einer Vielzahl fokussierter Ultraschallstrahlen bestehen, die jeweils eine entsprechende Sendefokusposition aufweisen, und wobei das empfangene Echosignal aus einer Vielzahl von Echosignalen als Reaktion auf die gesendete Vielzahl fokussierter Ultraschallstrahlen besteht.

5. Das Verfahren nach einem der vorstehenden Ansprüche, das weiter einen Schritt zum Filtern des Signalphasenkohärenzbildes umfasst.

6. Das Verfahren nach einem der vorstehenden Ansprüche, wobei der erste und der zweite Abschnitt der Vielzahl von Wandlerelementen identisch sind.

7. Das Verfahren nach einem der vorstehenden Ansprüche, das weiter einen Schritt zum Quantifizieren der Phasenkohärenz des Echosignals umfasst.

8. Das Verfahren nach Anspruch **4,** wobei die Vielzahl von Strahlen als parallele, gleichzeitige Strahlen gesendet werden.

9. Ein Ultraschall-Bildgebungssystem, umfassend:
- eine flexible Ultraschallvorrichtung, wobei die flexible Ultraschallvorrichtung eine flexible Trägerplattform und eine Vielzahl von Wandlerelementen umfasst, die in einer Array-Konfiguration der flexiblen Trägerplattform angeordnet sind;
- eine Datenspeichereinheit, die ein Formschätzungsmodul speichert und konfiguriert ist, um Daten aus dem Formschätzungsmodul zu speichern; und
- eine Datenverarbeitungseinheit, die mit der flexiblen Ultraschallvorrichtung und der Datenspeichereinheit verbunden ist, und angepasst ist, um Daten aus der flexiblen Ultraschallvorrichtung zu empfangen und um die empfangenen Daten zu verarbeiten;
wobei, wenn ausgeführt, das Formschätzungsmodul den Schritt durchführt zum:
- Senden unter Verwendung eines ersten Abschnitts der Vielzahl von Wandlerelementen und basierend auf einer angenommenen Form der flexiblen Ultraschallvorrichtung eines fokussierten Ultraschallstrahls, der eine vorbestimmte Sendefokusposition aufweist;
- Empfangen unter Verwendung eines zweiten Abschnitts der Vielzahl von Wandlerelementen eines Echosignals als Reaktion auf den gesendeten fokussierten Ultraschallstrahl, wobei jedes Wandlerelement des zweiten Abschnitts der Vielzahl von Wandlerelementen elektronisch mit einem jeweiligen Empfangskanal verbunden ist;
- Berechnen basierend auf dem empfangenen Echosignal eines Signalphasenkohärenzbildes über die Empfangskanäle; und
- Schätzen durch Berechnen eines räumlichen Versatzes im Signalphasenkohärenzbild zwischen der vorgegebenen Sendefokusposition und einem beobachteten Ort minimaler Phasenkohärenz der tatsächlichen Form der flexiblen Ultraschallvorrichtung.

10. Das Ultraschall-Bildgebungssystem nach Anspruch 9, wobei beim Ausführen des Formschätzungsverfahrens der räumliche Versatz einen axialen Versatz umfasst, um eine relative Weglänge der Elementpositionen je Element zu korrigieren, und einen seitlichen Versatz, um Winkelkoordinaten der Elemente in Bezug zum Scheitelpunkt zu korrigieren.

11. Das Ultraschallbildgebungssystem nach Anspruch 9, wobei beim Ausführen des Formschätzungsverfahrens der berechnete räumliche Versatz mit einem Kalibrierungsbild oder einer Kalibrierungskurve verglichen wird, um die tatsächliche Form der flexiblen Ultraschallvorrichtung zu schätzen.

12. Ein Computerprogramm, das Anweisungen umfasst, um das System nach einem der Ansprüche 9 bis 11 zu veranlassen, die Schritte des Verfahrens nach einem der Ansprüche 1 bis 8 auszuführen.

## Revendications

1. Un procédé pour estimer une forme réelle d'un dispositif à ultrasons flexible, dans lequel le dispositif à ultrasons flexible comprend une plateforme de support flexible et une pluralité d'éléments transducteurs disposés en configuration matricielle sur la plateforme de support flexible, le procédé comprenant :
- la transmission, en utilisant une première partie de la pluralité d'éléments transducteurs et basée sur une forme supposée du dispositif à ultrasons flexible, d'un faisceau d'ultrasons focalisé ayant une position de focalisation de transmission prédéterminée ;
- la réception, en utilisant une seconde partie de la pluralité d'éléments transducteurs, d'un signal d'écho en réponse au faisceau d'ultrasons focalisé transmis, dans lequel chaque élément transducteur de la seconde partie de la pluralité d'éléments transducteurs est connecté électroniquement à un canal de réception respectif ;
- le calcul, basé sur le signal d'écho reçu, d'une image de cohérence de phase du signal à travers les canaux de réception ; et
- l'estimation, par le calcul d'un décalage spatial dans l'image de cohérence de phase du signal entre la position de focalisation de transmission prédéterminée et un emplacement de cohérence de phase minimale observé, de la forme réelle du dispositif à ultrasons flexible.

2. Le procédé selon la revendication 1, dans lequel le décalage spatial comprend un décalage axial pour corriger les positions des éléments transducteurs par rapport à la longueur de chemin relative par élément transducteur, et un décalage latéral pour corriger les coordonnées angulaires des éléments transducteurs par rapport à un sommet du dispositif à ultrasons flexible.

3. Le procédé selon la revendication 1, dans lequel le décalage spatial calculé est comparé avec une image de calibration ou une courbe de calibration pour estimer la forme réelle du dispositif à ultrasons flexible.

4. Le procédé selon l'une quelconque des revendications précédentes, dans lequel les faisceaux d'ultrasons focalisés transmis consistent en une pluralité de faisceaux d'ultrasons focalisés ayant chacun une position de focalisation de transmission correspondante, et dans lequel le signal d'écho reçu consiste en une pluralité de signaux d'écho en réponse à la pluralité de faisceaux d'ultrasons focalisés transmis.

5. Le procédé selon l'une quelconque des revendications précédentes, comprenant en outre une étape de filtrage de l'image de cohérence de phase du signal.

6. Le procédé selon l'une quelconque des revendications précédentes, dans lequel la première et la seconde partie des éléments transducteurs sont identiques.

7. Le procédé selon l'une quelconque des revendications précédentes, comprenant en outre une étape de quantification de la cohérence de phase du signal d'écho.

8. Le procédé selon l'une quelconque des revendications précédentes, dans lequel la pluralité de faisceaux est transmise sous forme de faisceaux parallèles simultanés.

9. Un système d'imagerie par ultrasons, comprenant :
- un dispositif à ultrasons flexible, dans lequel le dispositif à ultrasons flexible comprend une plateforme de support flexible et une pluralité d'éléments transducteurs disposés en configuration matricielle sur la plateforme de support flexible ;
- une unité de stockage de données stockant un module d'estimation de forme et configurée pour stocker des données provenant du module d'estimation de forme ; et
- une unité de traitement de données connectée au dispositif à ultrasons flexible et à l'unité de stockage de données, et adaptée pour recevoir des données du dispositif à ultrasons flexible et pour traiter les données reçues ;
dans lequel, lorsqu'il est exécuté, le module d'estimation de forme effectue les étapes suivantes :
- la transmission, en utilisant une première partie de la pluralité d'éléments transducteurs et basée sur une forme supposée du dispositif à ultrasons flexible, d'un faisceau d'ultrasons focalisé ayant une position de focalisation de transmission prédéterminée ;
- la réception, en utilisant une seconde partie de la pluralité d'éléments transducteurs, d'un signal d'écho en réponse au faisceau d'ultrasons focalisé transmis, dans lequel chaque élément transducteur de la seconde partie de la pluralité d'éléments transducteurs est connecté électroniquement à un canal de réception respectif ;
- le calcul, basé sur le signal d'écho reçu, d'une image de cohérence de phase du signal à travers les canaux de réception ; et
- l'estimation, par le calcul d'un décalage spatial dans l'image de cohérence de phase du signal entre la position de focalisation de transmission prédéterminée et un emplacement de cohérence de phase minimale observé, de la forme réelle du dispositif à ultrasons flexible.

10. Le système d'imagerie par ultrasons selon la revendication 9, dans lequel, lors de l'exécution du procédé d'estimation de forme, le décalage spatial comprend un décalage axial pour corriger les positions des éléments par rapport à la longueur de chemin relative par élément, et un décalage latéral pour corriger les coordonnées angulaires des éléments par rapport au sommet.

11. Le système d'imagerie par ultrasons selon la revendication 9, dans lequel, lors de l'exécution du procédé d'estimation de forme, le décalage spatial calculé est comparé avec une image ou une courbe de calibration pour estimer la forme réelle du dispositif à ultrasons flexible.

12. Un programme informatique comprenant des instructions pour amener le système selon l'une quelconque des revendications 9 à 11 à exécuter les étapes du procédé selon l'une quelconque des revendications 1 à 8.
